# EUROPÄISCHE PATENTANMELDUNG

(11) **EP 1 209 155 A1**
(43) Veröffentlichungstag der Anmeldung: **29.05.2002**
(21) Anmeldenummer: 00125407.7
(22) Anmeldetag: 20.11.2000
(51) Int. Cl.: C07D 301/03, C07C 1/22

(54) **Koppelprodukt freies Verfahren zur Herstellung von Propylenoxid**

(71) Anmelder: Degussa AG, 40474 Düsseldorf (DE)
(72) Erfinder: Haas, Thomas, Dr., 60322 Frankfurt (DE); Thiele, Georg, Dr., 63450 Hanau (DE); Hofen, Willi, 63517 Rodenbach (DE)
(74) Vertreter: Sternagel, Fleischer, Godemeyer & Partner Patentanwälte

(57) **Zusammenfassung**

Die vorliegenden Erfindung betrifft ein Verfahren zur Herstellung von Propylenoxid aus Propen, molekularem Sauerstoff und Wasserstoff, bei dem kein Koppelprodukt auftritt , wobei das Verfahren die Schritte umfasst:
a) Oxidation eines Kohlenwasserstoffs mit einem sekundären oder tertiären Kohlenstoffatom mit molekularem Sauerstoff zum entsprechenden Hydroperoxid,
b) Umsetzung des Hydroperoxids aus Schritt a) mit Propen in Gegenwart eines Epoxidierungskatalysators zu Propylenoxid und dem Alkohol,
c) Abtrennen des Alkohols von gebildetem Propylenoxid und nicht umgesetztem Propen,
d) Hydrieren des in Schritt c) erhaltenen Alkohols mit Wasserstoff in Gegenwart eines Hydrierkatalysators zum Kohlenwasserstoff und
e) Rückführen des in Schritt d) erhaltenen Kohlenwasserstoffs in Schritt a).

## Beschreibung

Die Erfindung betrifft ein Koppelprodukt-freies Verfahren zur Herstellung von Propylenoxid aus Propylen, molekularem Sauerstoff und Wasserstoff.

### Stand der Technik:

Zur Herstellung von Propylenoxid werden in großem Maßstab sogenannte Hydroperoxidverfahren eingesetzt, bei denen ein Kohlenwasserstoff mit Luftsauerstoff zu einem Hydroperoxid oxidiert wird und anschließend Propen mit diesem Hydroperoxid in Gegenwart eines Epoxidierungskatalysators zu Propylenoxid umgesetzt wird. Bei diesen Verfahren wird aus dem Hydroperoxid der entsprechende Alkohol als Koppelprodukt zu Propylenoxid gebildet, der durch weitere Reaktionen in ein verkaufsfähiges Produkt umgesetzt wird. Bei der Verwendung von 2-Methylpropan (Isobutan) als Kohlenwasserstoff erhält man tert-Butanol, das durch Reaktion mit Methanol zu tert-Butylmethylether (MTBE) umgesetzt wird (PO/MTBE-Verfahren). Bei der Verwendung von Ethylbenzol als Kohlenwasserstoff erhält man 1-Phenylethanol, das durch Dehydratisierung zu Styrol umgesetzt wird (PO/SM-Verfahren). Die Verfahren sind in ihrer technischen Ausführung aus Ullmann's Encyclopedia of Industrial Chemistry 5^{th} Ed., Vol. A22, S. 239-260 bekannt.

Gemeinsames Merkmal der Hydroperoxidverfahren ist die Herstellung von Propylenoxid zusammen mit einem Koppelprodukt, das in größeren Mengen anfällt als Propylenoxid und im durch das Verfahren vorgegebenen Mengenverhältnis zu Propylenoxid vermarktet werden muss. Da sich der Bedarf an Propylenoxid und den Koppelprodukten nicht im gleichen Maß entwickelt, besteht ein Bedarf an Verfahren zur Herstellung von Propylenoxid ohne den Anfall eines Koppelprodukts. Aufgabe der vorliegenden Erfindung ist daher, ein Verfahren zur Herstellung von Propylenoxid zur Verfügung zu stellen, bei dem keine Koppelprodukte auftreten.

### Gegenstand der Erfindung:

Diese Aufgabe wird durch ein Verfahren zur Herstellung von Propylenoxid aus Propen, molekularem Sauerstoff und Wasserstoff gelöst, wobei das Verfahren die Schritte umfasst:
a) Oxidation eines Kohlenwasserstoffs mit einem sekundären oder tertiären Kohlenstoffatom mit molekularem Sauerstoff zum entsprechenden Hydroperoxid,
b) Umsetzung des Hydroperoxids aus Schritt a) mit Propen in Gegenwart eines Epoxidierungskatalysators zu Propylenoxid und dem Alkohol,
c) Abtrennen des Alkohols von gebildetem Propylenoxid und nicht umgesetztem Propen,
d) Hydrieren des in Schritt c) erhaltenen Alkohols mit Wasserstoff in Gegenwart eines Hydrierkatalysators zum Kohlenwasserstoff und
e) Rückführen des in Schritt d) erhaltenen Kohlenwasserstoffs in Schritt a.

Die Auswahl des Kohlenwasserstoffs in Schritt a) ist nicht kritisch solange der Kohlenwasserstoff über ein sekundäres oder tertiäres Kohlenstoffatom verfügt und die daraus resultierende C-H-Bindung sich durch molekularen Sauerstoff zum entsprechenden Hydroperoxid oxidieren lässt. Das erfindungsgemäße Verfahren wird bevorzugt mit Kohlenwasserstoffen der Formel R¹R²R³CH betrieben, worin R¹, R² und R³ unabhängig von einander aus substituierten oder unsubstituierten Alkyl-, Aryl-, Alkaryl-, Aralkyl-, Cycloalkylgruppen, und Wasserstoff ausgewählt sind, unter der Vorrausetzung, dass maximal einer von R¹, R² und R³ Wasserstoff sein kann und die Substituenten an R¹, R² und R³ so ausgewählt sind, dass diese weder mit der Oxidationsreaktion in Schritt a) noch mit der Hydrierung in Schritt d) wechselwirken. In einer bevorzugten Ausführungsform ist R¹ Phenyl oder Methyl, R² Methyl und R³ Methyl oder Wasserstoff. Besonders bevorzugt werden die Kohlenwasserstoffe 2-Methylpropan (Isobutan), Ethylbenzol und 1-Methylethylbenzol (Cumol) verwendet.

Schritt a) des erfindungsgemäßen Verfahrens kann in bekannter Weise durch die Umsetzung des Kohlenwasserstoffs mit molekularem Sauerstoff bei erhöhter Temperatur durchgeführt werden. Der Sauerstoff kann dabei sowohl in reiner Form, als auch in Form von Mischungen mit Inertgas, z.B. in Form von Luft eingesetzt werden. Die Umsetzung erfolgt vorzugsweise bei Temperaturen von 120 bis 170°C. Die Umsetzung wird vorzugsweise so geführt, dass nur ein Teil des Kohlenwasserstoffs zum Hydroperoxid umgesetzt wird und eine Lösung des Hydroperoxids im Kohlenwasserstoff erhalten wird. Die so erhaltene Lösung des Hydroperoxids kann durch Verdampfen von nicht umgesetztem Kohlenwasserstoff aufkonzentriert werden, bevor sie in Schritt b) des Verfahrens eingesetzt wird.

In Schritt b des erfindungsgemäßen Verfahrens wird in bekannter Weise das in Schritt a) hergestellte Hydroperoxid mit Propen in Gegenwart eines Epoxidierungskatalysators zu Propylenoxid und dem Alkohol, vorzugsweise R¹R²R³COH umgesetzt, wobei R¹, R² und R³ wie oben definiert sind. Als Epoxidierungskatalysator eignen sich Molybdänverbindungen die sich in der Reaktionsmischung homogen lösen. Alternativ können auch heterogene Katalysatoren eingesetzt werden, bei denen Titan auf einem Kieselsäureträger fixiert ist. Die Epoxidierung wird vorzugsweise bei Temperaturen von 80 bis 130°C mit einem molaren Überschuss an Propen durchgeführt. Katalysatormenge und Verweilzeit werden so gewählt, dass ein Hydroperoxidumsatz von mehr als 95%, vorzugsweise mehr als 98% erzielt wird. Die Reaktion kann optional in Gegenwart eines Lösungsmittels durchgeführt werden, wobei als Lösungsmittel bevorzugt der für die Hydroperoxidherstellung verwendete Kohlenwasserstoff und/oder der aus dem Hydroperoxid entstehende Alkohol verwendet werden.

In Schritt c) des erfindungsgemäßen Verfahrens wird der in Schritt b) aus dem Hydroperoxid entstandene Alkohol von gebildetem Propylenoxid und nicht umgesetztem Propen abgetrennt. Für die Abtrennung können alle geeigneten physikalischen Trennverfahren eingesetzt werden. Bevorzugt erfolgt die Abtrennung durch Destillation, wobei Propen und Propylenoxid mit dem Kopfprodukt und der Alkohol mit dem Sumpfprodukt erhalten werden. Falls in Schritt b) ein löslicher Epoxidierungskatalysator eingesetzt wird, wird dieser im Anschluss an die Abtrennung von Propen und Propylenoxid ebenfalls von dem Alkohol abgetrennt, wobei die Wahl des Trennverfahrens nach den Eigenschaften des Alkohols erfolgt. Bei wasserlöslichen Alkoholen mit niedrigem Siedepunkt, wie z.B. tert-Butanol erfolgt die Abtrennung eines Epoxidierungskatalysators vorzugsweise durch Destillation, wobei der Alkohol mit dem Kopfprodukt erhalten wird. Bei wenig wasserlöslichen Alkoholen, wie z.B. 1-Phenylethanol oder Cumylalkohol erfolgt die Abtrennung eines Epoxidierungskatalysators vorzugsweise durch Extraktion mit einer alkalischen wässrigen Lösung, in die der Epoxidierungskatalysator extrahiert wird.

In Schritt d) des erfindungsgemäßen Verfahrens wird der in Schritt c) abgetrennte Alkohol, vorzugsweise R¹R²R³COH mit molekularem Wasserstoff in Gegenwart eines Hydrierkatalysators zum Kohlenwasserstoff, vorzugsweise R¹R²R³CH, wobei R¹, R² und R³ wie oben definiert sind umgesetzt. Als Hydrierkatalysatoren werden bevorzugt die Metalle Ni, Ru, Rh, Pd, Os, Ir und Pt der 8. Nebengruppe des Periodensystems eingesetzt, sowie Verbindungen dieser Elemente, die unter den Reaktionsbedingungen mit Wasserstoff zum Metall reagieren. Besonders bevorzugt werden die Metalle Pd und Ru als Hydrierkatalysatoren verwendet. Die Metalle werden vorzugsweise in Form von Trägerkatalysatoren eingesetzt, in denen das Metall auf einem inerten Trägermaterial aufgebracht ist. Geeignete Trägermaterialien sind Aktivkohle und Metalloxide wie Aluminiumoxid, Siliziumoxid, Titanoxid oder Aluminium-Silizium-Mischoxide. Die Hydrierung wird bei einer Temperatur im Bereich von 30°C bis 250°C durchgeführt, wobei die Temperatur je nach der Reaktionsfähigkeit des Alkohols und der Aktivität des Hydrierkatalysators gewählt wird. Bevorzugt wird die Hydrierung bei erhöhten Temperaturen im Bereich von 100°C bis 250°C durchgeführt. Der Wasserstoff wird unter einem erhöhtem Druck von 10 bis 300 bar eingesetzt. Die Reaktion kann optional in Gegenwart eines oder mehrerer Lösungsmittel durchgeführt werden, wobei vorzugsweise Wasser als Lösungsmittel eingesetzt wird. Die Reaktionsdauer wird vorzugsweise so gewählt, dass mehr als 20%, besonders bevorzugt mehr als 50% des Alkohols umgesetzt werden. Die Reaktion wird vorzugsweise so durchgeführt, dass der Alkohol, optional zusammen mit einem Lösungsmittel, in flüssiger Phase vorliegt und der Hydrierkatalysator entweder in der flüssigen Phase suspendiert wird oder die flüssige Phase über ein Festbett geleitet wird, das den Hydrierkatalysator enthält. Der für die Reaktion benötigte Wasserstoff wird gasförmig eingesetzt. Bei Verwendung eines suspendierten Hydrierkatalysators wird der Wasserstoff vorzugsweise durch eine geeignete Dispergiereinrichtung, wie z.B. einen Begasungsrührer oder eine Düse in der Flüssigphase verteilt. Bei Verwendung eines Festbettkatalysators wird der Wasserstoff im Gegenstrom oder im Gleichstrom zur Flüssigphase durch den Reaktor geleitet, wobei der Reaktor sowohl als Rieselbettreaktor mit kontinuierlicher Gasphase als auch als Blasensäulenreaktor mit kontinuierlicher Flüssigphase betrieben werden kann.

Die Rückführung des in Schritt d) erhaltenen Kohlenwasserstoffs in Schritt a) des Verfahrens kann in der Regel, nachdem falls verwendet der suspendierte Hydrierungskatalysator abgetrennt worden ist, ohne weitere Reinigungsstufen erfolgen. Falls in Schritt d) ein Lösungsmittel verwendet wird, kann dieses jedoch optional vor Schritt e) des erfindungsgemäßen Verfahrens abgetrennt und in die Hydrierung von Schritt d) zurückgeführt werden.

Das erfindungsgemäße Verfahren hat den entscheidenden Vorteil, dass hiermit Propylenoxid ohne irgend ein Koppelprodukt zugänglich ist und somit die Produktion einfach der Nachfrage an Propylenoxid angepasst werden kann, ohne dass der Markt für das Koppelprodukt berücksichtigt werden muss. Dies führt zu einer Verbesserung der Wirtschaftlichkeit des Verfahrens.

Das erfindungsgemäße Verfahren weist den zusätzlichen Vorteil auf, dass bestehende Anlagen zur Herstellung von Propylenoxid nach dem Hydroperoxidverfahren, die neben Propylenoxid ein Koppelprodukt erzeugen, durch das Ergänzen um den Hydrierschritt aus Schritt d) des integrierten Verfahrens so umgerüstet werden können, dass Propylenoxid ohne den Zwangsanfall eines Koppelprodukts hergestellt werden kann. Somit können bereits bestehende Anlagen mit vergleichsweise geringen Investitionskosten in einfacher Weise umgerüstet werden.

In den folgenden Beispielen soll die vorliegende Erfindung noch verdeutlicht werden.

### Beispiel 1: Hydrogenolyse von tert-Butanol

Es werden 1750 g tert-Butanol, 1750 g Wasser und 17,5 g 3 % Palladium auf Aktivkohle-Katalysator in einen 5 l Autoklaven gegeben. Bei Raumtemperatur wird ein Druck von 20 bar Wasserstoff eingestellt und anschließend das Reaktionsgemisch auf 180°C erwärmt. Nach einer Reaktionszeit von 1 h wird der Autoklav wieder abgekühlt, der Katalysator abfiltriert und eine repräsentative Probe der flüssigen und Gasphase entnommen. Der Umsatz an tert-Butanol betrug 60 %, in der flüssigen Phase waren neben Wasser und tert-Butanol noch 2 % Nebenprodukte detektiert worden. In der Gasphase wurde neben Wasserstoff nur Isobutan gefunden. Daraus ergibt sich eine Reaktionsselektivität von 96,6 %.

### Beispiel 2: Hydrogenolyse von tert-Butanol

Der Versuch wurde wie in Beispiel 1 durchgeführt mit der Ausnahme, dass 17,5 g eines Katalysators 5 % Ruthenium auf Aluminiumoxid eingesetzt wurde. Der Umsatz an tert-Butanol betrug 98 %, in der Flüssigphase wurde, bezogen auf eingesetztes tert-Butanol, noch 10 % Nebenprodukte gefunden. In der Gasphase wurde neben Wasserstoff nur Isobutan detektiert. Daraus ergibt sich eine Selektivität von 89,8 %.

### Beispiel 3: Hydrogenolyse von 2-Propanol

Es wurden 1.750 g 2-Propanol, 1.750 g Wasser, 17,5 g 5 % Ruthenium auf Aluminiumoxid in einem 5-l-Autoklaven gemischt. Bei Raumtemperatur wurden 50 bar Wasserstoff aufgedrückt und anschließend das Reaktionsgemisch auf 220°C erwärmt. Die Reaktionsdauer betrug 1 h. Danach wurde der Autoklav wieder abgekühlt. Eine repräsentative Probe der flüssigen und Gasphase wurde entnommen. 20 % des 2-Propanol wurden umgesetzt. In der Flüssigphase wurden, bezogen auf das eingesetzte 2-Propanol, 3 % Nebenprodukte detektiert. In der Gasphase wurde neben Wasserstoff nur Propan gefunden. Daraus ergibt sich eine Reaktionsselektivität von 85 %.

### Beispiel 4: Hydrogenolyse von 2-Propanol

Der Versuch wurde wie in Beispiel 3 durchgeführt mit dem Unterschied, dass kein Wasser, sondern nur 3.500 g 2-Propanol eingesetzt wurde. Der Umsatz betrug 40 %. In der Flüssigphase wurden, bezogen auf eingesetztes 2-Propanol, 10 % Nebenprodukte detektiert, in der Gasphase nur Propan neben Wasserstoff. Daraus ergibt sich eine Reaktionsselektivität von 75 %.

## Patentansprüche

1. Verfahren zur Herstellung von Propylenoxid aus Propen, molekularem Sauerstoff und Wasserstoff, wobei das Verfahren die Schritte umfasst:
a) Oxidation eines Kohlenwasserstoffs mit einem sekundären oder tertiären Kohlenstoffatom mit molekularem Sauerstoff zum entsprechenden Hydroperoxid,
b) Umsetzung des Hydroperoxids aus Schritt a) mit Propen in Gegenwart eines Epoxidierungskatalysators zu Propylenoxid und dem Alkohol,
c) Abtrennen des Alkohols von gebildetem Propylenoxid und nicht umgesetztem Propen,
d) Hydrieren des in Schritt c) erhaltenen Alkohols mit Wasserstoff in Gegenwart eines Hydrierkatalysators zum Kohlenwasserstoff und
e) Rückführen des in Schritt d) erhaltenen Kohlenwasserstoffs in Schritt a).

2. Verfahren nach Anspruch 1, wobei als Hydrierkatalysator in Schritt d) ein Metall der 8. Nebengruppe eingesetzt wird.

3. Verfahren nach einem der vorstehend Ansprüche, wobei der Hydrierkatalysator auf einem Träger angeordnet ist.

4. Verfahren nach Anspruch 3, wobei der Hydrierkatalysator als Festbett in dem in Schritt d) verwendeten Hydrierreaktor angeordnet ist.

5. Verfahren nach einem der vorstehend Ansprüche, wobei die Hydrierung in Schritt d) in einem Lösungsmittel durchgeführt wird.

6. Verfahren nach Anspruch 5 wobei dieses Lösungsmittel vor Schritt e) von dem Kohlenwasserstoff abgetrennt wird und zu Schritt d) zurückgeführt wird.

7. Verfahren nach einem der Ansprüche 5 und 6, wobei das Lösungsmittel Wasser ist.

8. Verfahren nach einem der vorstehenden Ansprüche, wobei der Kohlenwasserstoffen der Formel R¹R²R³CH entspricht, worin R¹, R² und R³ unabhängig von einander aus substituierten oder unsubstituierten Alkyl-, Aryl-, Alkaryl-, Aralkyl-, Cycloalkylgruppen, und Wasserstoff ausgewählt sind, unter der Vorrausetzung, dass maximal einer von R¹, R² und R³ Wasserstoff sein kann und die Substituenten an R¹, R² und R³ so ausgewählt sind, dass diese weder mit der Oxidationsreaktion in Schritt a) noch mit der Hydrierung in Schritt d) wechselwirken.

9. Verfahren nach Anspruch 8, wobei R¹ Phenyl oder Methyl, R² Methyl und R³ Methyl oder Wasserstoff ist.

10. Verfahren nach Anspruch 9, wobei der Kohlenwasserstoff in Schritt a) aus 2-Methylpropan, Ethylbenzol und 1-Methylethylbenzol ausgewählt ist.
